# EUROPÄISCHE PATENTSCHRIFT

(11) **EP 2 792 739 B1**
(45) Veröffentlichungstag und Bekanntmachung des Hinweises auf die Patenterteilung: **24.03.2021**
(21) Anmeldenummer: 13001963.1
(22) Anmeldetag: 15.04.2013
(51) Int. Cl.: C12M 1/107, C12M 1/113, C12M 1/26, C12M 1/33

(54) **Biogasanlage mit einer Fermenter-Beschickungseinrichtung**
Biogas plant with a fermenter feeding device
Installation de biogaz dotée d'un dispositif d'alimentation en ferments

(43) Veröffentlichungstag der Anmeldung: 22.10.2014
(73) Patentinhaber: BTS Biogas Srl/GmbH, 39031 Bruneck (IT)
(72) Erfinder: Niederbacher, Michael, 39031 Bruneck (IT)
(74) Vertreter: Liebl, Thomas

(56) Entgegenhaltungen:
- EP-A- 0 201 928
- EP-A- 0 555 792
- EP-A- 0 613 870
- EP-A- 1 125 894
- EP-A- 1 978 086
- EP-A1- 0 781 742
- WO-A-2005/028375
- WO-A-2008/029163
- WO-A2-2010/086158
- DE-A1- 3 844 700
- DE-A1- 10 121 034
- DE-A1-102007 029 700
- GB-A- 1 212 680
- US-A1- 2005 026 262
- US-A1- 2011 262 985

## Beschreibung

Die Erfindung betrifft eine Biogasanlage mit einer Fermenter-Beschickungseinrichtung zur Zuführung von Biomasse in einen Fermenterbehälter der Biogasanlage nach dem Oberbegriff des Anspruchs 1. Aus der gattungsbildenden WO 2010/086158 A2 ist bereits eine Fermenter-Beschickungsanlage für vergärbare Biomasse einer Biogasanlage bekannt, die eine Förder- und Aufbereitungseinrichtung zwischen einer Biomasselagerstelle und einem Fermenter bzw. Fermenterbehälter der Biogasanlage aufweist. Diese Förder- und Aufbereitungseinrichtung weist ein Fördermittel für die dem Fermenter zuzuführende Biomasse auf, wobei in die Förder- und Aufbereitungseinrichtung weiter eine mechanische Zellaufschlussvorrichtung für Biomasse in Form eines Zellaufschlussextruders integriert ist, über die zumindest ein Teil der zum Fermenter förderbaren Biomasse geleitet und nach dem Zellaufschluss dem Fermenter zugeführt wird. Konkret kann hier der Durchmesser einer Auslassöffnung des Zellaufschlussextruders durch ein gesteuert verstellbares Stellglied, zum Beispiel elektrisch, hydraulisch oder pneumatisch verändert werden, wobei ein Temperatursensor bzw. ein Drucksensor vorgesehen ist, mittels dem die Temperatur und/oder der Druck in einem vorgegebenen Bereich des Zellaufschlussextruders bzw. der Biomasse erfasst wird. Ferner ist ein Durchmesser-Regler vorgesehen, dem das Ausgangssignal des Temperatursensors als Temperatur-Istwert bzw. das Ausgangssignal des Drucksensors als Druck-Istwert zugeführt wird, wobei der Istwert mit einem vorgegebenen Sollwert verglichen wird und das Stellglied bei einem Absinken des Istwerts unter den Sollwert für eine vorgegebene Reduzierung des Auslassöffnungs-Durchmessers angesteuert wird.

Durch einen solchen Zellaufschluss können vorteilhaft Biomassefeststoffe bei der Biogaserzeugung im Fermentationsprozess, zum Beispiel Stroh und Gras verwendet werden, die ansonsten nicht bzw. nur uneffektiv vergärbar sind. Weiter tritt in Verbindung mit dem Zellaufschluss eine Zerkleinerung der Biomassefeststoffe auf, so dass diese für die Bakterien im Fermenter und damit im Gärprozess besser zugängig sind bzw. eine leichter angreifbare Struktur ausbilden, so dass ein hoher Trockensubstanzgehalt im Substrat bei geringen Verweilzeiten mit hoher Biogasausbeute möglich ist. Zudem können dadurch bei der Dimensionierung und beim Betrieb der Fermenter-Rührtechnik Einsparungen erzielt werden, wobei eine Tendenz zur Bildung von Schwimmschichten oder Sinkschichten reduziert wird. Durch diese Vorbehandlung mittels eines Zellaufschlussextruders kann die Biogasausbeute der Biomasse somit erheblich gesteigert werden, was wiederum einen geringen Einsatz an Biomasse zur Folge hat und was sich wiederum in kleineren Baugrößen für die Fermenter wiederspiegelt, so dass die Biogasanlage in der Anschaffung und auch im Betrieb kostengünstiger ist als herkömmliche Biogasanlagen.

Mit der weiter vorgesehenen Temperatur- bzw. Druckregelung des Durchmessers kann zudem erreicht werden, dass bei einer definierten gleichen Leistungsaufnahme des Zellaufschlussextruders mehr Energie in die aktuell darin geförderte und aufbereitete Biomasse eingebracht wird, so dass eine auf den jeweiligen Einzelfall abgestimmte optimale Biomasse für eine Vergärung in einem Fermenter einer Biogasanlage zur Verfügung gestellt werden kann.

Im praktischen Betrieb hat es sich jedoch insbesondere bei faserigen Biomassefeststoffen als nachteilig erwiesen, dass sich die Fasern um die Schneckenwellen des Zellaufschlussextruders herumwickeln und dadurch den Extrusionsprozess beeinträchtigen können. Insbesondere kann es aufgrund der dortigen hohen Temperaturen sogar unter Umständen zu einem Anbacken der zu extrudierenden Biomasse an den Schneckenwellen kommen. Dies kann dann wiederum insgesamt die Zuführung von Biomasse in einen Fermenterbehälter einer Biogasanlage beeinträchtigen.

Die weitere EP 0 781 742 A1 offenbart einen Extruder mit wenigstens einer Schleifstange, die in den Innenraum des Extrudergehäuses hineinragt. Diese Schleifstange ist, in Extruder-Förderrichtung gesehen, stromab einer Schneckenwendelung einer Schneckenwelle des Extruders und stromauf einer Zerkleinerungsplatte des Extruders angeordnet. Mittels dieser Schleifstange soll die Rotationsbewegung des mittels des Extruders geförderten Materials verringert werden, bevor das Material zu der stromab der Schnecke des Extruders angeordneten Zerkleinerungsplatte gefördert wird.

Die GB 1 212 680 A betrifft eine Fördereinrichtung, mittels der sperriger Abfall in einen Extruder gefördert werden kann. Diese Fördereinrichtung weist einen verschwenkbaren Arm auf, der in einer definierten Schwenkposition, in Extruder-Förderrichtung gesehen, stromauf der Schneckenwendelung der Schneckenwelle angeordnet ist.

Aus der DE 101 21 034 A1 geht eine Vorrichtung zum kontinuierlichen Dosieren von Textil- und Folienschnipsel sowie Fasermaterial zur Weiterverarbeitung in einem Doppelschneckenextruder hervor, bei der die Textil- und Folienschnipsel und das Fasermaterial mittels zweier parallel liegender, gegenläufiger Dosierwalzen aus dem unteren Ende eines Vorratsbehälters abgezogen werden. Jede der Dosierwalzen ist hier als Stiftwalze ausgebildet, die in Dosierwalzen-Axialrichtung voneinander beabstandete Stiftreihen mit mehreren radial von einer Dosierwalzen-Welle abragende Stifte aufweist. Die Dosierwalzen sind dabei derart angeordnet, dass die Stiftreihen der Dosierwalzen miteinander kämmen. Des Weiteren sind hier Abstreifelemente vorgesehen, mittels denen zwischen den Stiftreihen der Dosierwalzen angesammeltes Material von den Dosierwalzen entfernt werden kann. Die Abstreifelemente sind durch Zinken ausgebildet, die in die Zwischenräume zwischen den Stiftreihen hineinragen.

Aufgabe der vorliegenden Erfindung ist es, eine Biogasanlage mit einer Fermenter-Beschickungseinrichtung zur Zuführung von Biomasse in einen Fermenterbehälter der Biogasanlage zur Verfügung zu stellen, mittels der eine Beschickung eines oder mehrerer Fermenterbehälter unter Verwendung eines Zellaufschlussextruders auf funktionssichere Weise ohne Beeinträchtigung des Beschickungsvorgangs durchgeführt werden kann.

Diese Aufgabe wird gelöst mit den Merkmalen des Anspruchs 1. Vorteilhafte Ausgestaltungen sind Gegenstand der darauf rückbezogenen Unteransprüche.

Gemäß Anspruch 1 ist eine Biogasanlage mit einer Fermenter-Beschickungseinrichtung zur Zuführung von Biomasse in einen Fermenterbehälter der Biogasanlage vorgesehen, wobei die Fermenter-Beschickungseinrichtung einen Zellaufschlussextruder aufweist, der wenigstens eine, in einem Extrudergehäuse aufgenommene Schneckenwelle mit einer erhabenen Schneckenwendelung aufweist, und wobei der Zellaufschlussextruder wenigstens eine Zuführeinrichtung für zu extrudierenden Biomasse sowie wenigstens eine Auslassöffnung für eine extrudierte Biomasse aufweist. Erfindungsgemäß ist im Innenraum des Schneckengehäuses wenigstens eine Räumeinrichtung angeordnet, die sich über wenigstens einen Teilbereich der Länge einer zugeordneten Schneckenwelle erstreckt, wobei die Räumeinrichtung mehrere in Schneckenwellen-Längsrichtung voneinander beabstandete Räumzinken aufweist, wobei die Räumzinken mit ihren freien Zinkenenden bis zu der zugeordneten Schneckenwelle geführt sind. Weiter erfindungsgemäß weist die erhabene Schneckenwendelung in jedem Durchgangsbereich eines Räumzinkens durch die erhabene Schneckenwendelung eine Zinkenausnehmung auf, die von dem jeweils zugeordneten Räumzinken bei einer Schneckenwellenbetätigung bzw. -drehung durchgriffen wird, insbesondere dergestalt durchgriffen wird, dass Biomassefeststoffe, die sich auf der Schneckenwelle befinden und nicht mehr weiter extrudiert bzw. weiter transportiert werden können, von der Schneckenwelle abgeschabt und/oder entfernt werden können.

Mit einer derartigen erfindungsgemäßen Lösung wird somit auf einfache Weise sichergestellt, dass um die Schneckenwelle herumgewickelte faserige Biomassefeststoffe bzw. an der Schneckenwelle angebackene Biomassefeststoffe zuverlässig von der Schneckenwelle abgeschabt bzw. entfernt werden können, so dass es zu keinem Verstopfen des Extruders bzw. zu keiner Beeinträchtigung des Extrusionsprozesses durch sich dort ansammelnde Biomassefeststoffe kommen kann. Dadurch wird somit die Freigängigkeit der wenigstens einen Schneckenwelle des Zellaufschlussextruders sichergestellt, was sich vorteilhaft auf den gesamten Extrusionsprozess auswirkt, der somit ungehindert ablaufen kann, wodurch die gewünschte funktionssichere Beschickung eines Fermenterbehälters mit aufgeschlossener Biomasse zu vorgegebenen Zeiten in vorgegebener Menge möglich wird. Dadurch wird die Funktionssicherheit im Hinblick auf das Betreiben einer Biogasanlage insgesamt erhöht. Zudem wird durch die mehreren in Schneckenwellen-Längsrichtung voneinander beabstandeten Räumzinken die Effektivität des Abschab- bzw. Räumvorgangs erhöht.

Es versteht sich, dass die Räumzinken aus einem stabilen Material hergestellt sein müssen, zum Beispiel aus einem Stahlmaterial, zum Beispiel aus einem Edelstahlmaterial.

Wie erfinderseitige Versuche gezeigt haben, ergibt sich eine besonders zuverlässige und funktionssichere Entfernung von Biomassefeststoffen dann, wenn die schneckenwendelungsseitige Zinkenausnehmung gemäß einer besonders bevorzugten Ausgestaltung so ausgebildet ist, dass diese von dem jeweils zugeordneten Räumzinken bei einer Schneckenwellendrehung mit einem definierten Spaltabstand form- und/oder konturangepasst durchgriffen wird. Mit einem derartigen form- und/oder konturangepassten Durchgriff wird somit der Schab-, Räum- und/oder Schneidvorgang begünstigt, weil dann der Räumzinken mit seinem freien Zinkenende mit der zugeordneten Zinkenausnehmung bzw. deren die Zinkenausnehmung ausbildenden Randkanten eine Art Schlagleisten- oder Schlagkantenanordnung ausbildet.

Der wenigstens eine Räumzinken ist gemäß einer weiteren besonders bevorzugten Ausgestaltung mit seinem freien Zinkenende so zur zugeordneten Schneckenwelle geführt, dass das freie Zinkenende dort mit wenigstens einen Teilbereich anliegt oder das freie Zinkenende dort von der Schneckenwelle einen definierten Spaltabstand aufweist. Dies umfasst ausdrücklich auch den Fall, dass das freie Zinkenende dort mit einem Teilbereich anliegt und mit einem weiteren Teilbereich einen definierten Spaltabstand aufweist. Besonders bevorzugt ist dabei eine Ausgestaltung, bei der der Spaltabstand zwischen Zinkenende und Schneckenwelle, vorzugsweise in Drehrichtung der Welle gesehen, zunimmt, insbesondere sich sichelförmig erweitert. Mit einer derartigen unmittelbaren Zuordnung des freien Zinkenendes zur Schneckenwelle wird sichergestellt, dass bei einer entsprechenden Betätigung bzw. Drehung der Schneckenwelle auch bereits dünnste Schichten abgeschabt werden können bzw. sich eng um die Schneckenwelle legende Fasern aufgefranst und abgezogen bzw. aufgeschnitten werden können, was wesentlich dazu beiträgt, die Schneckenwelle insgesamt von störenden Biomassefeststoffschichten freizuhalten. Eine derartige konkrete Zuordnung des freien Zinkenendes zu der Schneckenwelle führt somit auf besonders zuverlässige Weise zu dem gewünschten Räumergebnis. Der sich in Drehrichtung der Welle erweiternde, insbesondere sich sichelförmig erweiternde Spaltabstand weist dabei zusätzlich den Vorteil auf, dass lediglich eine Schneidkante bzw. eine Schabkante des freien Sichelendes in unmittelbarer Nähe zur Schneckenwelle liegt bzw. dort mehr oder weniger anliegt, so dass die Hauptwirkung auf die Schneidwirkung gelegt wird und sichergestellt wird, dass sich zwischen Zinkenende und Welle keine unerwünschte Verklemmung ergeben kann.

Besonders bevorzugt ist eine Ausgestaltung, bei der der wenigstens eine Räumzinken in seinem Abstand zur zugeordneten Schneckenwelle und/oder in seiner Länge verstellbar ausgebildet ist. Dies erlaubt ein einfaches Nachjustieren bzw. Nachstellen bei zum Beispiel einem Verschleiß an den Räumzinken. Mit anderen Worten kann bei dieser Ausführungsvariante der wenigstens eine Räumzinken zur Einstellung eines definierten Spaltmaßes zwischen dem wenigstens einen Räumzinken und der zugeordneten Schneckenwelle einfachst und individuell, das heißt bei mehreren Räumzinken gegebenenfalls auch unterschiedlich weit, in Richtung auf die zugeordnete Schneckenwelle zu oder von dieser weg verlagert werden. Zudem begünstigt bzw. ermöglicht diese Variante einen einfachen Austausch von zum Beispiel verschlissenen Räumzinken. Die Verstellung kann zum Beispiel durch Schraubverstellung mit Gewinde, bei der der Räumzinken mit einem Außengewinde mehr oder weniger weit in eine zugeordnete Gewindeaufnahme eingeschraubt wird, oder aber auch auf jede andere Weise erfolgen.

Der Abschab-, Räum- bzw. Schneidvorgang wird zudem weiter dadurch unterstützt, dass gemäß einer weiteren besonders bevorzugten Ausgestaltung vorgesehen ist, dass wenigstens eine der die Zinkenausnehmung ausbildenden Randkanten der Schneckenwendelung und/oder das Zinkenende des der jeweiligen Zinkenausnehmung zugeordneten Räumzinkens wenigstens eine Schneid- und/oder Schlagkante aufweist bzw. ausbildet. Gemäß einer besonders bevorzugten konkreten Ausgestaltung kann die wenigstens eine Schneid- und/oder Schlagkante keilförmig und/oder spitz zulaufend ausgebildet sein. Alternativ oder zusätzlich dazu kann die Schneid- und/oder Schlagkante aber auch durch eine stufenartige bzw. zerklüftete Oberflächenstruktur gebildet sein, beispielsweise auch durch entsprechende Aufschweißungen bzw. Einsätze zusätzlich schneidend bzw. reißend verstärkt und/oder ausgebildet sein.

Das Schab- bzw. Schneidergebnis wird zudem noch dadurch weiter verbessert, dass die wenigstens eine Schneid- und/oder Schlagkante gemäß einer weiteren besonders bevorzugten Ausgestaltung an der wenigstens einen Randkante einer Zinkenausnehmung und/oder an einem Zinkenende des jeweils zugeordneten Räumzinkens im Wesentlichen quer zur Drehachse der wenigstens einen Schneckenwelle ausgerichtet ist. Dadurch wird auf einfache Weise sichergestellt, dass die Schneidkanten quer zur Extruder-Förderrichtung verlaufen und damit ein besonders gutes Schab- und Schneidergebnis erzielt werden kann.

Es ist des Weiteren vorteilhaft, dass die Zuführeinrichtung wenigstens eine am Extrudergehäuse ausgebildete Einwurföffnung aufweist, über die zu extrudierende Biomasse dem Zellaufschlussextruder zuführbar ist. In diesem Zusammenhang ist weiter bevorzugt vorgesehen, dass oberhalb der Einwurföffnung ein Einwurfschacht angeordnet ist. Damit wird ein kontrolliertes Zuführen von zu extrudierender Biomasse in den Extruder möglich. Besonders bevorzugt weist der Einwurfschaft einen oberen Einwurftrichter und ein sich an den Einwurftrichter nach unten zur Einwurföffnung hin anschließendes sowie sich nach unten konisch erweiterndes Schachtrohr auf, mittels denen die Zuführung begünstigt wird. Vorzugsweise kann im Bereich des Schachtrohres zudem eine Revisionsöffnung mitsamt zugeordneten Deckel, Klappe oder dergleichen vorgesehen sein.

Die wenigstens eine Räumeinrichtung und damit der wenigstens eine Räumzinken ist zudem bevorzugt in einem der Einwurföffnung zugewandten Schneckenwellenbereich angeordnet, weil dort die Wahrscheinlichkeit einer Brückenbildung oder das Vorhandensein von nicht aufgeschlossener, langfasriger Biomassefeststoffe am kritischsten ist und somit dort die Gefahr eines Verstopfens des Extruders besteht.

Besonders bevorzugt ist weiter eine Ausgestaltung, bei der im Bereich der Einwurföffnung ein vorzugsweise erhabenes Leitelement, vorzugsweise in Form eines Prismas, angeordnet ist, das sich im wesentlichen in Querrichtung und/oder über einen definierten Teilbereich im Bereich der Einwurföffnung erstreckt. Mittels eines derartigen Leitelementes kann auf funktionssichere Weise vermieden werden, dass die Biomasse beim Vorwärtsfördern mittels der wenigstens einen Schneckenwelle gegebenenfalls wieder nach oben in den Bereich der Einwurföffnung gelangt. Aus diesem Grund ist bevorzugt vorgesehen, dass das Leitelement an einer Randkante der Einwurföffnung und/oder an einem der Einwurföffnung zugewandten Randkantenbereich des Einwurfschachtes und/oder wenigstens an einem in Förderrichtung gesehen stromabwärtigen vorderen Teilbereich der Einwurföffnung angeordnet ist.

Um insbesondere eine Brückenbildung im Bereich der Einwurföffnung zu vermeiden ist bevorzugt vorgesehen, dass der wenigstens eine Räumzinken so in Hochachsenrichtung gesehen von der Einwurföffnung weg nach unten versetzt ist, dass dieser einen in Hochachsenrichtung gesehen definierten Mindestabstand von der Einwurföffnung aufweist.

Gemäß einer besonders bevorzugten Ausgestaltung erstreckt sich der wenigstens eine Räumzinken wenigstens mit einem das freie Zinkenende aufweisenden Teilbereich, vorzugsweise insgesamt, in etwa in gerader Verlängerung entlang des von der Wellendrehachse ausgehenden Radius der Schneckenwelle von der Schneckenwelle weg. Damit wird auf einfache Weise eine besonders stabile und funktionssichere Anordnung der Räumzinken sichergestellt, weil der Kraftfluss beim Schab- bzw. Räumvorgang dann direkt entlang der Radiusrichtung über den Räumzinken zum Beispiel in das Gehäuse eingeleitet und somit dort funktionssicher und zuverlässig abgestützt werden kann. Bei einer demgegenüber mehr winkligen Anordnung, bei der sich der Räumzinken von der Schneckenwelle weg nicht in gerader Verlängerung des Radius erstreckt, ergeben sich ungünstigere Kraftflussverhältnisse und muss dementsprechend der Räumzinken bzw. die Räumeinrichtung entsprechend dimensioniert werden.

Der erfindungsgemäße Zellaufschlussextruder kann grundsätzlich ein Extruder mit einer einzigen Schneckenwelle sein. Besonders bevorzugt ist jedoch eine Ausgestaltung, bei der der Zellaufschlussextruder für hervorragende Extrusionsergebnisse ein Doppelschneckenextruder mit zwei Schneckenwellen ist. Insbesondere in Verbindung mit einem derartigen Doppelschneckenextruder mit zwei Schneckenwellen ist es von Vorteil, dass sich von, mit Bezug auf die nebeneinanderliegenden Schneckenwellen gegenüberliegenden Gehäusewänden, insbesondere Gehäuseseitenwänden bei in Querrichtung nebeneinanderliegenden Schneckenwellen, jeweils wenigstens eine Räumeinrichtung mit ihrem wenigstens einen Räumzinken nach innen in Richtung zu einer der beiden Schneckenwellen erstreckt. Durch diese Ausgestaltung ist dann auf einfache Weise sichergestellt, dass jeder der beiden Schneckenwellen eine bzw. mehrere erfindungsgemäße Räumeinrichtungen zugeordnet sind, so dass die Biomassefeststoffe zuverlässig von beiden Schneckenwellen abgeschabt bzw. geräumt werden können.

Für eine besonders funktionssichere Extrusion und Zuführung der Biomasse in den Eingriffsbereich der beiden aufeinander zudrehenden Schneckenwellen ist vorgesehen, dass auf einer Seite dieses Eingriffsbereichs ein gehäuseseitiges Leitelement angeordnet ist, mit dem die zu extrudierende Biomasse in Richtung Eingriffsbereich der beiden Schneckenwellen geleitet werden kann. Ebenso kann auf der gegenüberliegenden "auswerfenden" Seite des Eingriffsbereichs ein derartiges Leitelement vorgesehen sein, das eine Führungsfunktion für die aus dem Eingriffsbereich kommende Biomasse ausübt.

Die Räumeinrichtung bzw. der wenigstens eine Räumzinken kann grundsätzlich integral, das heißt einstückig und/oder materialeinheitlich, mit dem Gehäuse ausgebildet sein. Besonders bevorzugt ist die wenigstens eine Räumeinrichtung jedoch lösbar mit dem Gehäuse verbunden, um zum Beispiel bei einem Verschleiß der Räumeinrichtung bzw. der Räumzinken diese austauschen zu können. Für die lösbare Festlegung am Gehäuse weist die Räumeinrichtung vorzugsweise eine Trägerplatte auf, die mit der Gehäuseinnenwand lösbar verbunden werden kann, zum Beispiel mittels einer festen Schraubverbindung verbunden werden kann, wobei von einer solchen Trägerplatte dann der wenigstens eine Räumzinken in der gewünschten Weise abragt. Auch der Räumzinken kann entweder integral mit der Trägerplatte verbunden oder aber mit dieser entsprechend verbunden sein.

Insbesondere für Wartungsarbeiten und/oder zum Austragen der abgeschabten bzw. abgeschnittenen Biomasse von der Schneckenwelle kann vorgesehen sein, dass der wenigstens eine, mit der wenigstens einen Räumeinrichtung versehene Schneckenbereich über mit einem Verschlusselement, insbesondere mit einem Deckel oder einer Klappe verschließbare Revisions- bzw. Zugangsöffnung von außerhalb des Schneckengehäuses zugänglich ist.

Die Erfindung wird nachfolgend anhand einer Zeichnung näher erläutert.

Es zeigen:
- Fig. 1: schematisch eine Biogasanlage mit einer Fermenter-Beschickungsanlage,
- Fig. 2: eine perspektivische Ansicht einer beispielhaften Ausführungsform eines Zellaufschlussextruders,
- Fig. 3: schematisch eine vergrößerte Darstellung der Einzelheit A der Fig. 2,
- Fig. 4: schematisch eine Draufsicht auf den Extruder gemäß Fig. 2,
- Fig. 5: eine vergrößerte Darstellung der Einzelheit C der Fig. 4,
- Fig. 6: eine Schnittansicht entlang der Linie B-B der Fig. 4,
- Fig. 7: eine vergrößerte Darstellung der Einzelheit D der Fig. 6,
- Fig. 8: schematisch eine Seitenansicht des Zellaufschlussextruders mit einem über einer einer Einwurföffnung angeordnetem Einwurfschacht,
- Fig. 9: schematisch eine Schnittansicht entlang der Linie A-A der Fig. 8,
- Fig. 10: schematisch eine vergrößerte Darstellung der Einzelheit B der Fig. 8, und
- Fig. 11: schematisch eine perspektivische Darstellung des Zellaufschlussextruders mit lediglich teilweise aufgesetztem Einwurfschacht.

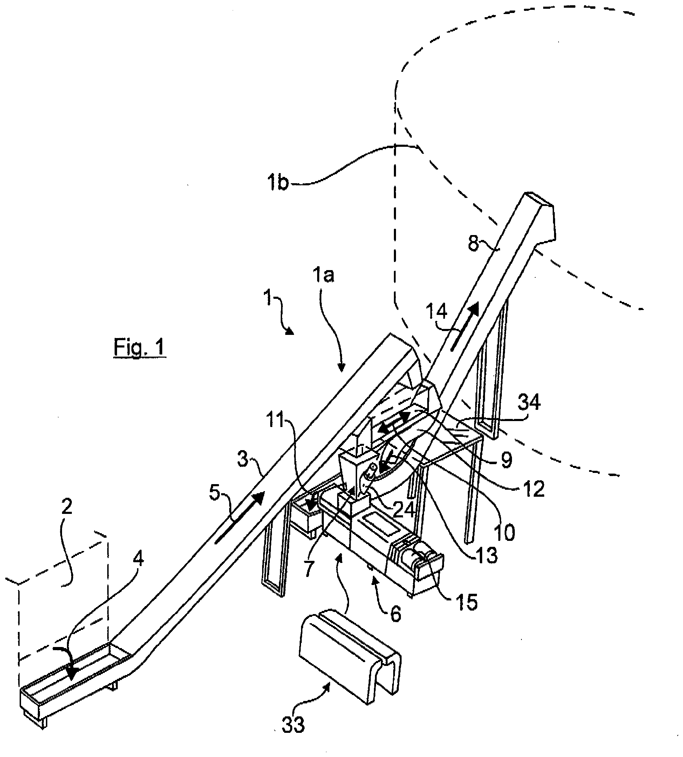

In der Fig. 1 ist eine Biogasanlage 1 mit einer Fermenter-Beschickungseinrichtung 1a sowie einem hier lediglich schematisch und strichliert dargestellten Fermenterbehälter 1b gezeigt, wobei einer (nicht dargestellten) Biomasselagerstelle, zum Beispiel als Fahrsilo, ein nur schematisch und strichliert angedeuteter Dosierer 2 nachgeordnet ist.

Vom Dosierer 2 wird gewichtsgesteuert Biomasse einem ersten Bandförderer 3 zugeführt (Pfeil 4), mit dem die Biomasse nach oben gefördert wird (Pfeil 5), und zwar in einem Bereich oberhalb eines Zellaufschlussextruders 6. Dort ist unter dem oberen Ende des ersten Bandförderers 3 und über eine Zuführeinrichtung 7 des Zellaufschlussextruders 6 ein mit einem zweiten Bandförderer 8 unmittelbar oder mit der Zuführeinrichtung 7 zusammenwirkender, dritter Bandförderer 9 angebracht. Dazu kann der dritte Bandförderer 9 in der Förderrichtung umgesteuert werden (Doppelpfeil 10), so dass (im Normalbetrieb) Biomasse, die vom ersten Bandförderer 3 auf den dritten Bandförderer 9 transportiert wird, über die Zuführeinrichtung 7 dem Zellaufschlussextruder 6 und nach der dortigen Aufbereitung dem zweiten Bandförderer 8 zugeführt wird (Pfeil 11). Bei einer Umsteuerung der Laufrichtung des dritten Bandförderers 9, beispielsweise nach einer automatisierten Erkennung von Störstoffen im Förderweg zum Zellaufschlussextruder 6, gelangt Biomasse über ein Förderrohr 12 in einen Direktweg als Bypass durch freien Fall im Bereich des Bandanfangs des zweiten Bandförderers 8 (Pfeil 13).

Mit dem zweiten Bandförderer 8 wird aufbereitete Biomasse aus dem Zellaufschlussextruder 6 (Pfeil 11) über den Direktweg (Pfeil 13) nach oben transportiert (Pfeil 14), so dass dann letztendlich die aufgeschlossene Biomasse aus dem Zellaufschlussextruder 6 in den Fermenterbehälter 1b gefördert werden kann.

Dieser Aufbau in der Fig. 1 ist lediglich beispielhaft. Selbstverständlich ist es möglich, auch andere Ausführungen vorzusehen, mittels denen von einem Zellaufschlussextruder 6 aufgeschlossene Biomasse in einen Fermenterbehälter gefördert werden kann.

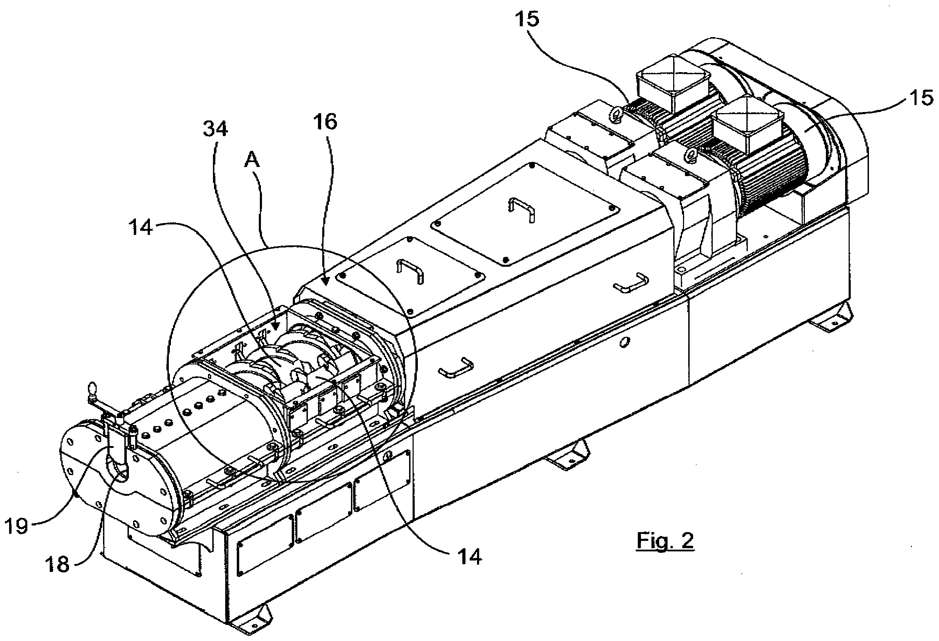

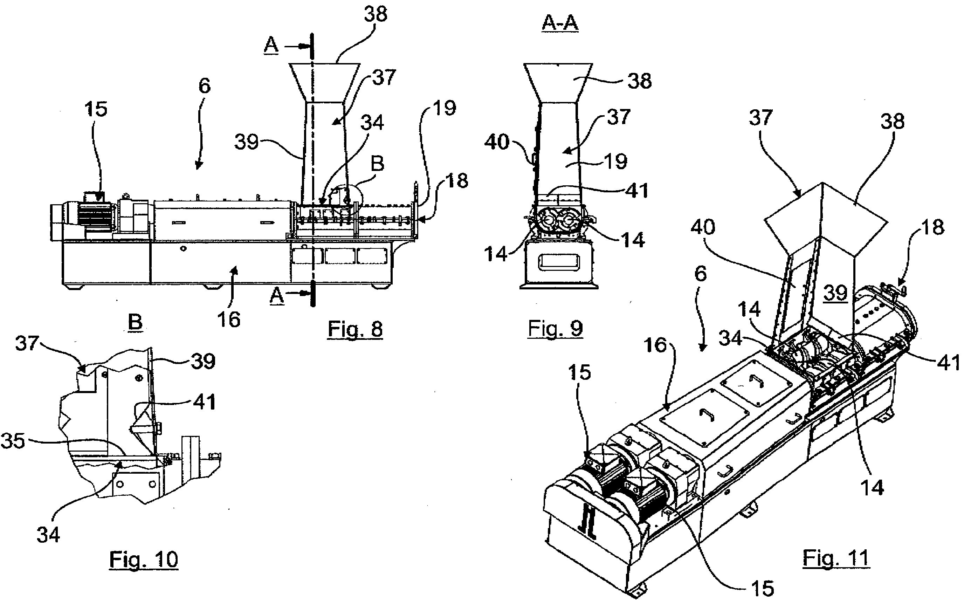

In der Fig. 2 ist nunmehr schematisch und perspektivisch der hier beispielhaft als Doppelschneckenextruder mit zwei Schneckenwellen 14 ausgebildete Zellaufschlussextruder 6 gezeigt, wobei die beiden Schneckenwellen 14 jeweils durch einen Elektromotor 15 angetrieben werden. Die beiden Schneckenwellen 14 sind in einem Schneckengehäuse 16 des Zellaufschlussextruders 6 aufgenommen, das gegebenenfalls zusätzlich wärmegedämmt ist, was hier aber nicht dargestellt ist. Das Gehäuse ist hier lediglich zur Erläuterung einer beispielhaften Ausführungsform der im Innenraum des Schneckengehäuses 16 angeordneten Räumeinrichtungen 17 teilweise offen dargestellt. Wie dies nachfolgend noch in Verbindung mit den Fig. 8 bis 11 näher erläutert wird, wird über dieser hier gezeigten Einwurföffnung 34 ein Einwurfschacht 37 angeordnet.

Der Fig. 2 kann desweiteren entnommen werden, dass der Zellaufschlussextruder 6 an einem vorderen Ende eine Auslassöffnung 18 mit regelbaren Schieberklappe 19 aufweist, über die die jeweils gewünschte Menge an extrudierter Biomasse aus dem Schneckengehäuse 16 ausgebracht wird. Insofern entspricht der Zellaufschlussextruder 6 demjenigen, wie er in der WO 2010/086158 A2 beschrieben worden ist.

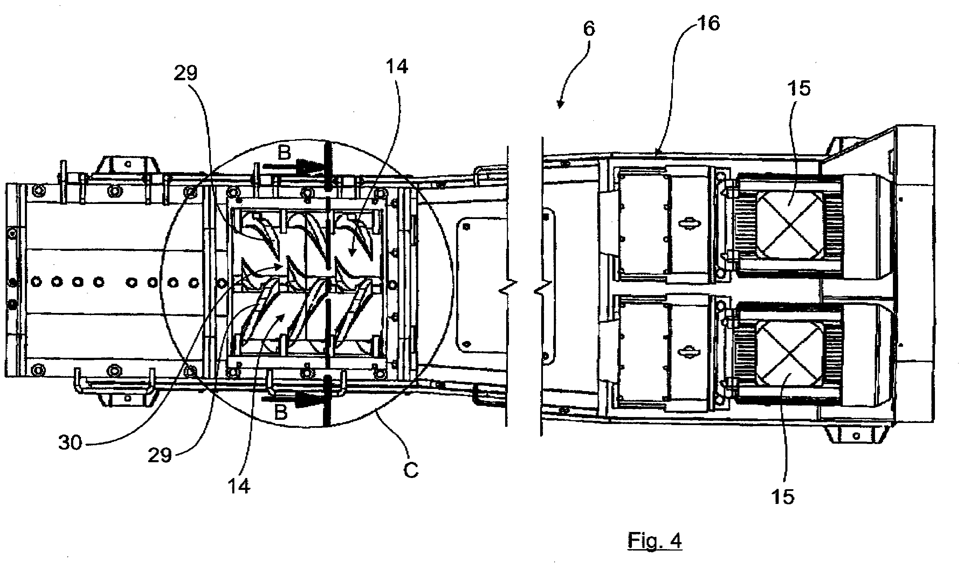

Anders als bei der WO 2010/086158 A2 ist jedoch, was insbesondere aus der Zusammenschau der Fig. 2 bis 7 sehr gut ersichtlich ist, im Innenraum des Schneckengehäuses 16 jeweils zu beiden Seiten der in Querrichtung nebeneinanderliegenden Schneckenwellen 14 jeweils eine Räumeinrichtung 17 mit mehreren voneinander beabstandeten Räumzinken 20 angeordnet. Wie dies insbesondere aus der Fig. 2 und 4 hervorgeht, sind die Räumeinrichtungen 17 auf den gegenüberliegenden Gehäusesseitenwänden 21, 22 im Wesentlichen identisch aufgebaut und erstrecken sich im auslassöffnungsnahen Bereich über lediglich einen Teilbereich der Schneckenwellenlänge.

Die hier beispielhaft jeweils vier Räumzinken 20 pro Räumeinrichtung 17 sind beispielsweise auf einer Trägerplatte 23 gehaltert, die mit der Innenwand des Schneckengehäuses 16 zum Beispiel kraftschlüssig mittels mehrerer Schraubverbindungen lösbar verbunden sind.

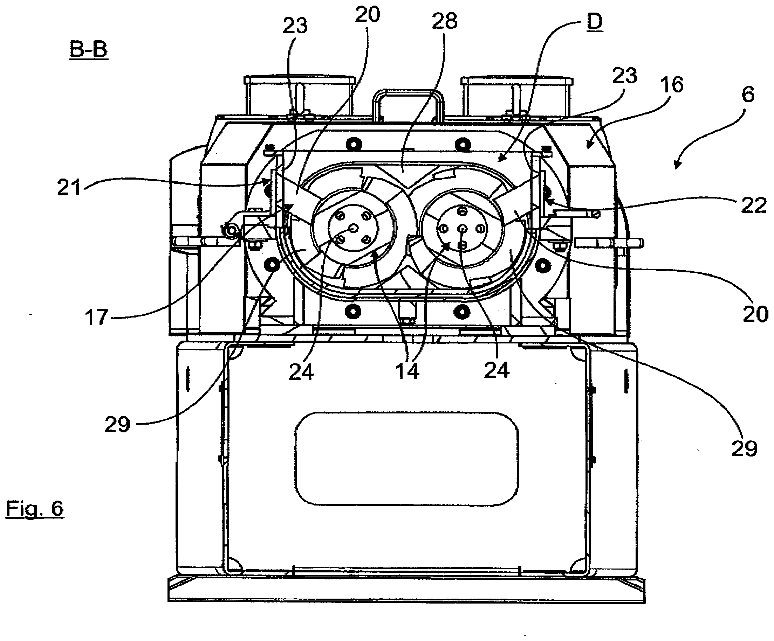

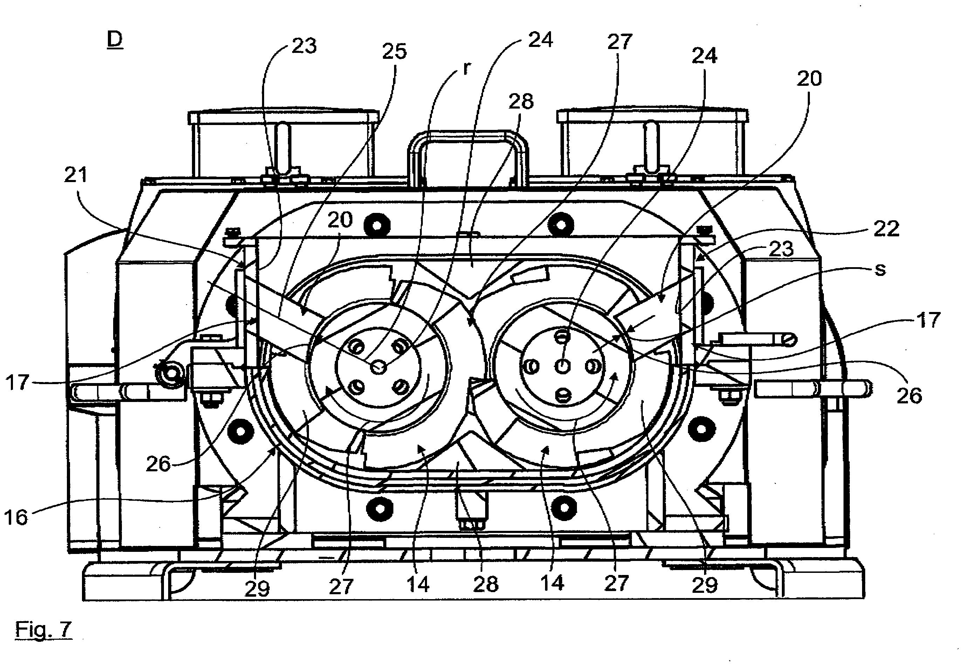

Wie dies insbesondere aus der Fig. 6 und 7 ersichtlich ist, erstrecken sich die Räumzinken 20 jeweils in etwa in gerader Verlängerung entlang des von der Wellendrehachse 24 bzw. vom Wellenmittelpunkt ausgehenden Radius r der Schneckenwellen 14 von der jeweiligen Schneckenwelle 14 weg. Dies ist in der Fig. 7 schematisch durch die gerade Linie 25 eingezeichnet.

Wie dies der Fig. 6 und insbesondere der Fig. 7 zudem weiter entnommen werden kann, sind die Räumzinken 20 zudem mit ihren freien Zinkenenden 26 so zur jeweils zugeordneten Schneckenwelle 14 geführt, dass das freie Zinkenende 26 dort von der Schneckenwelle 14 einen definierten Spaltabstand s aufweist, wobei sich dieser Spaltabstand s jeweils in Drehrichtung (Pfeil 27) der Schneckenwellen 14 sichelförmig erweitert.

Die Räumzinken 20 sind hier bevorzugt in ihrem Abstand zur jeweils zugeordneten Schneckenwelle 14 und/oder in ihrer Länge verstellbar ausgebildet, insbesondere dergestalt, dass die Räumzinken 20 zur Einstellung des definierten Spaltmaßes s in Richtung auf die zugeordnete Schneckenwelle 14 zu oder von dieser weg verlagerbar sind, wie dies in der Fig. 2 lediglich schematisch durch den Doppelpfeil 20a eingezeichnet ist.

Wie dies weiter insbesondere den Fig. 6 und 7 zu entnehmen ist, sind im Eingriffsbereich 27 der beiden aufeinander zudrehenden Schneckenwellen 14, hier beispielhaft zu beiden Seiten des Eingriffsbereichs 27, das heißt hier oberhalb und unterhalb des Eingriffsbereichs 27, jeweils gehäuseseitige Leitelemente 28 angeordnet, von denen das in der Bildebene obere Leitelement 28 die zu extrudierende Biomasse jeweils in Richtung Eingriffsbereich 27 der beiden Schneckenwellen 14 leitet, während das in der Bildebene untere Leitelement 28 das Abziehen der vom Eingriffsbereich 27 kommenden Biomasse unterstützt.

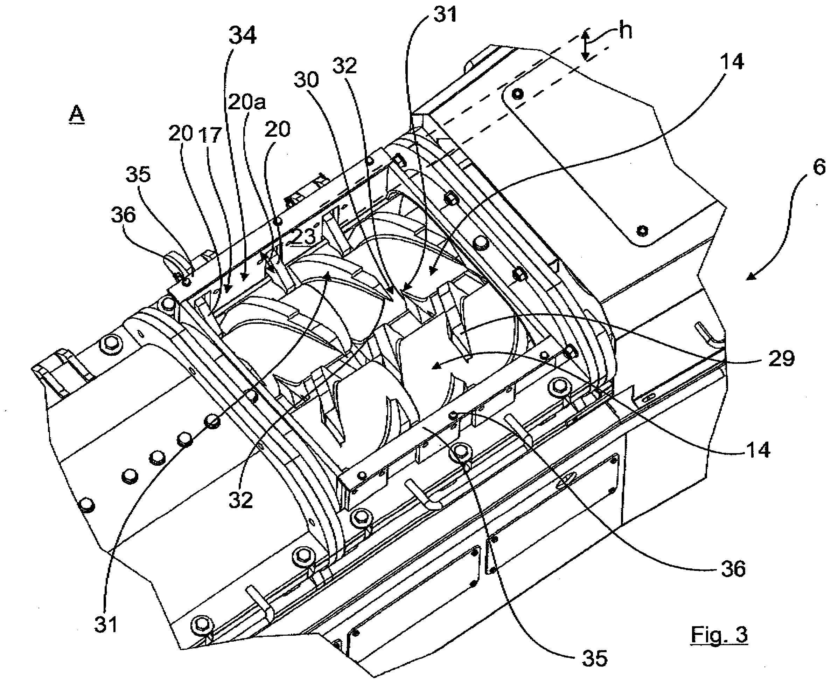

Zudem sind, was in der Fig. 3 lediglich äußert schematisch dargestellt ist, die Räumzinken 20 so in Hochachsenrichtung gesehen von dem die Einwurföffnung 34 ausbildenden Randbereich, der hier beispielhaft durch den Randflansch 35 gebildet ist, weg nach unten versetzt, dass diese einen in Hochachsenrichtung gesehen definiert vorgegebenen Mindestabstand h von der Einwurföffnung 34 aufweist, was hilft eine Brückenbildung im Bereich der Einwurföffnung 34 zu vermeiden.

Wie dies nunmehr insbesondere der Zusammenschau der Fig. 2 bis 5 entnommen werden kann, weisen die Schneckenwellen 14 jeweils eine sich schneckenförmig um den Wellenkörper erstreckende, erhabene Schneckenwendelung 29 auf, die in jedem Durchgangsbereich eines Räumzinkens 20 durch diese eine Zinkenausnehmung 30 aufweist, die bei einer Schneckenwellendrehung von dem jeweils zugeordneten Räumzinken 20 durchgriffen wird, so dass dann Biomassefeststoffe, die sich um die Schneckenwelle 14 gewickelt haben bzw. an dieser festgebacken sind, mittels der Räumzinken 20 von der Schneckenwelle 24 abgeschabt und entfernt werden können.

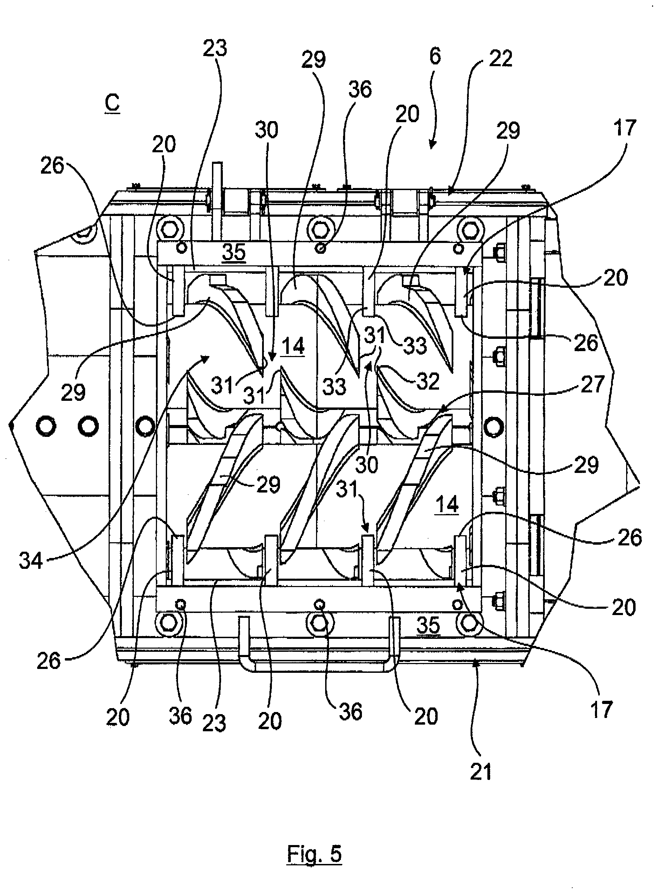

Die schneckenwendelungsseitige Zinkenausnehmung 30 ist dabei so ausgebildet, dass diese von den jeweils zugeordneten Räumzinken 20 bei einer Schneckenwellendrehung bevorzugt mit einem definierten geringen Spaltabstand form- und/oder konturangepasst durchgriffen wird, was die Schab- und Scherwirkung noch weiter erhöht. Dies geht insbesondere aus der Fig. 5 hervor, die deutlich zeigt, dass die Breite der Zinkenausnehmung 30 in etwa der Breite des Räumzinkens 20 entspricht bzw. leicht größer ist (vergleiche insbesondere untere Bildhälfte der Fig. 5).

Wie dies insbesondere den Fig. 3 und 5 entnommen werden kann, sind insbesondere die Zinkenausnehmungen 30 bzw. die die Zinkenausnehmung 30 ausbildenden Randkanten 31 mit einer keilförmig spitz zulaufenden Schneide 32 ausgebildet, die beim Vorbeigang der zinkenseitigen Schneidkanten 33 eine besonders vorteilhafte Scher- bzw. Schneidwirkung auf dort eventuell vorhandene Biomasse ausübt, so dass diese zuverlässig von der Schneckenwelle 24 abgeschabt bzw. abgeschnitten und somit entfernt werden kann.

Wie aus der Fig. 5 ersichtlich ist, sind die Schneiden 32 bzw. die Schneidkanten 33 hier hinsichtlich ihrer Wirkrichtung im Wesentlichen quer zur Wellendrehachse 24 der Schneckenwellen 14 ausgerichtet, was eine vorteilhafte Schneid- bzw. Scherbewegung zur Folge hat.

In den Fig. 8 bis 11 ist nunmehr gezeigt, dass im Bereich oberhalb der Einwurföffnung 34 ein Einwurfschacht 37 aufgesetzt sein kann, der bevorzugt mittels eines schachtseitigen Flansches mit dem Randflansch 35 der Einwurföffnung 34 in an sich üblicher Weise verbunden wird. Dieser Einwurfschacht 37 weist einen oberen Einwurftrichter 38 auf, an den sich nach unten zur Einwurföffnung 34 hin ein konisch erweiterndes Schachtrohr 39 anschließt, wobei, wie dies in der Fig. 9 und 11 ersichtlich ist, für einen Zugang zum Schneckenwellenbereich beziehungsweise zum Einwurföffnungsbereich eine Revisionsöffnung 40 ausgebildet sein kann, die hier mittels einer Tür verschlossen ist. Durch eine derartige geometrische Gestaltung des Einwurfschachts 37 mit einem oberen trichterförmigen Konus und einem sich daran nach unten hin anschließenden, das heißt zur Einwurföffnung 34 verbreitenden unteren Konus wird eine vorteilhafte Zudosierung der zu extrudierenden Biomasse zum Zellaufschlussextruder 6 ermöglicht.

Wie dies nunmehr insbesondere der Zusammenschau der Fig. 8 bis 11 entnommen werden kann, ist, hier beispielhaft am unteren schachtseitigen Randbereich, ein hier als gerades Prisma ausgebildetes erhabenes Leitelement 41 vorgesehen, beispielsweise mit der Schachtwand verschraubt. Dieses erhabene Leitelement 41 liegt in Strömungsrichtung der zu extrudierenden Biomasse gesehen stromabwärts in Richtung zur Auslassöffnung 18 hin, das heißt somit in Förderrichtung der Biomasse und erstreckt sich dabei hier beispielhaft über die gesamte Querwandseite des Einwurfschachtes 37. Mittels eines derartige zusätzlichen erhabenen Leitelements 41 kann auf einfache und funktonssichere Weise vermieden werden, dass die nach vorne zur Auslassöffnung 18 hin geförderte Biomasse gegebenenfalls wieder nach oben in den Bereich des Einwurfschachts 37 gelangen kann. Eine derartige Maßnahme trägt somit wesentlich dazu bei, die Funktionssicherheit beim Betrieb des Extruders 6 zu erhöhen.

## Patentansprüche

1. Biogasanlage mit einer Fermenter-Beschickungseinrichtung (1a) zur Zuführung von Biomasse in einen Fermenterbehälter (1b) der Biogasanlage (1), wobei die Fermenter-Beschickungseinrichtung (1a) einen Zellaufschlussextruder (6) aufweist, der wenigstens eine, in einem Extrudergehäuse (16) aufgenommene Schneckenwelle (14) mit einer erhabenen Schneckenwendelung (29) aufweist, und wobei der Zellaufschlussextruder (6) wenigstens eine Zuführeinrichtung für zu extrudierende Biomasse sowie wenigstens eine Auslassöffnung (18) für extrudierte Biomasse aufweist,
**dadurch gekennzeichnet,**
**dass** im Innenraum des Extrudergehäuses (16) wenigstens eine Räumeinrichtung (17) angeordnet ist, die sich über wenigstens einen Teilbereich der Länge einer zugeordneten Schneckenwelle (14) erstreckt, wobei die Räumeinrichtung (17) mehrere in Schneckenwellen-Längsrichtung voneinander beabstandete Räumzinken (20) aufweist, wobei die Räumzinken (20) mit ihren freien Zinkenenden (26) der zugeordneten Schneckenwelle (14) zugeordnet sind, und wobei die erhabene Schneckenwendelung (29) in jedem Durchgangsbereich eines Räumzinkens (20) durch die erhabene Schneckenwendelung (29) eine Zinkenausnehmung (30) aufweist, die von dem jeweils zugeordneten Räumzinken (20) bei einer Schneckenwellendrehung durchgriffen wird.

2. Biogasanlage nach Anspruch 1, **dadurch gekennzeichnet, dass** die schneckenwendelungsseitige Zinkenausnehmung (30) so ausgebildet ist, dass diese von dem jeweils zugeordneten Räumzinken (20) bei einer Schneckenwellendrehung mit einem definierten Spaltabstand form- und/oder konturangepasst durchgriffen wird.

3. Biogasanlage nach Anspruch 1 oder 2, **dadurch gekennzeichnet, dass** der wenigstens eine Räumzinken (20) mit seinem freien Zinkenende (26) der zugeordneten Schneckenwelle (14) so zugeordnet ist, dass das freie Zinkende (26) dort mit wenigstens einem Teilbereich anliegt oder das freie Zinkende (26) dort von der Schneckenwelle (14) einen definierten Spaltabstand (s) aufweist.

4. Biogasanlage nach Anspruch 3, **dadurch gekennzeichnet, dass** der Spaltabstand (s) zwischen Zinkenende (26) und Schneckenwelle (14), vorzugsweise in Drehrichtung der Schneckenwelle (14) gesehen, zunimmt, insbesondere sich sichelförmig erweitert.

5. Biogasanlage nach einem der vorhergehenden Ansprüche, **dadurch gekennzeichnet, dass** der wenigstens eine Räumzinken (20) in seinem Abstand zur zugeordneten Schneckenwelle (14) und/oder in seiner Länge verstellbar ausgebildet ist, insbesondere dergestalt, dass der wenigstens eine Räumzinken (20) zur Einstellung eines definierten Spaltmaßes (s) zwischen dem wenigstens einen Räumzinken (20) und der zugeordneten Schneckenwelle (14) in Richtung auf die zugeordnete Schneckenwelle (14) zu oder von dieser weg verlagerbar ist.

6. Biogasanlage nach einem der vorhergehenden Ansprüche, **dadurch gekennzeichnet, dass** wenigstens eine der die Zinkenausnehmung (30) ausbildenden Randkanten (31) der Schneckenwendelung (29) und/oder das Zinkenende (26) des der jeweiligen Zinkenausnehmung (30) zugeordneten Räumzinkens (20) wenigstens eine Schneid- und/oder Schlagkante (32, 33) aufweist und/oder ausbildet.

7. Biogasanlage nach Anspruch 6, **dadurch gekennzeichnet, dass** die wenigstens eine Schneid- und/oder Schlagkante (32, 33) keilförmig und/oder spitz zulaufend ausgebildet ist und/oder durch eine stufenartige und/oder zerklüftete Oberflächenstruktur gebildet ist.

8. Biogasanlage nach Anspruch 6 oder 7, **dadurch gekennzeichnet, dass** die wenigstens eine Schneid- und/oder Schlagkante (32, 33) an der wenigstens einen Randkante (31) einer Zinkenausnehmung (30) und/oder an einem Zinkenende (26) des jeweils zugeordneten Räumzinkens (20) im Wesentlichen quer zur Drehachse (24) der wenigstens einen Schneckenwelle (14) ausgerichtet ist oder sind.

9. Biogasanlage nach einem der vorhergehenden Ansprüche, **dadurch gekennzeichnet, dass** die Zuführeinrichtung wenigstens eine am Extrudergehäuse (16) ausgebildete Einwurföffnung (34) aufweist, über die zu extrudierende Biomasse dem Zellaufschlussextruder (6) zuführbar ist, wobei bevorzugt vorgesehen ist, dass oberhalb der Einwurföffnung (34) ein Einwurfschacht (37) angeordnet ist, der vorzugsweise einen oberen Einwurftrichter (38) und ein sich an den Einwurftrichter (38) nach unten zur Einwurföffnung (34) hin anschließendes sowie sich nach unten konisch erweiterndes Schachtrohr (39), vorzugsweise mit einer Revisionsöffnung (40), aufweist.

10. Biogasanlage nach Anspruch 9, **dadurch gekennzeichnet, dass** die wenigstens eine Räumeinrichtung (17) und damit der wenigstens eine Räumzinken (20) in einem der Einwurföffnung (18) zugewandten Schneckenwellenbereich angeordnet ist.

11. Biogasanlage nach Anspruch 9 oder 10, **dadurch gekennzeichnet, dass** im Bereich der Einwurföffnung (34), insbesondere an einer Randkante der Einwurföffnung (34) und/oder an einem der Einwurföffnung (34) zugewandten Randkantenbereich des Einwurfschachtes (37) und/oder wenigstens an einem in Förderrichtung gesehen stromabwärtigen vorderen Teilbereich der Einwurföffnung (34), ein Leitelement (41), vorzugsweise in Form eines Prismas, angeordnet ist, das sich im wesentlichen in Querrichtung und/oder über einen definierten Teilbereich im Bereich der Einwurföffnung (34) erstreckt.

12. Biogasanlage nach einem der Ansprüche 9 bis 11, **dadurch gekennzeichnet, dass** der wenigstens eine Räumzinken (20) so in Hochachsenrichtung gesehen von der Einwurföffnung (34) weg nach unten versetzt ist, dass dieser einen in Hochachsenrichtung gesehen definierten Mindestabstand von der Einwurföffnung (34) aufweist.

13. Biogasanlage nach einem der vorhergehenden Ansprüche, **dadurch gekennzeichnet, dass** sich der wenigstens eine Räumzinken (20) wenigstens mit einem das freie Zinkenende (26) aufweisenden Teilbereich in etwa in gerader Verlängerung entlang des von der Wellendrehachse (24) ausgehenden Schneckenwellenradius (r) von der Schneckenwelle (14) weg erstreckt.

14. Biogasanlage nach einem der vorhergehenden Ansprüche, **dadurch gekennzeichnet, dass** der Zellaufschlussextruder (6) ein Doppelschneckenextruder mit zwei Schneckenwellen (14) ist, wobei bevorzugt vorgesehen ist, dass sich von, mit Bezug auf die nebeneinanderliegenden Schneckenwellen (14), gegenüberliegenden Gehäusewänden (21, 22), insbesondere Gehäuseseitenwänden bei in Querrichtung nebeneinanderliegenden Schneckenwellen (14), jeweils wenigstens eine Räumeinrichtung (17) mit ihrem jeweils wenigstens einen Räumzinken (20) nach innen in Richtung zu einer der beiden Schneckenwellen (14) erstreckt.

15. Biogasanlage nach Anspruch 14, **dadurch gekennzeichnet, dass** im Eingriffsbereich (27) der beiden aufeinander zu drehenden Schneckenwellen (14) auf wenigstens einer Seite dieses Eingriffsbereichs (27) gehäuseseitige Leitelemente (28) angeordnet sind, mit denen die zu extrudierende Biomasse, je nach Lage und Drehrichtung, in Richtung Eingriffsbereich (27) der beiden Schneckenwellen (14) oder von diesem weg leitbar ist.

16. Biogasanlage nach einem der vorhergehenden Ansprüche, **dadurch gekennzeichnet, dass** die wenigstens eine Räumeinrichtung (17) einstückig oder lösbar mit dem Extrudergehäuse (16) verbunden ist, insbesondere eine Trägerplatte (23) aufweist, die mit der Gehäuseinnenwand lösbar verbindbar ist und von der der wenigstens eine Räumzinken (20) abragt.

## Claims

1. Biogas plant with a fermenter feeding device (1a) for feeding biomass into a fermenter tank (1b) of the biogas plant (1), wherein the fermenter feeding device (1a) has a cell breakdown extruder (6), which has at least one screw shaft (14), which is held in an extruder barrel (16) and has a raised screw helix (29), and wherein the cell breakdown extruder (6) has at least one feeding device for biomass to be extruded and also at least one outlet opening (18) for extruded biomass,
**characterized**
**in that** in the interior space of the extruder barrel (16) there is arranged at least one clearing device (17), which extends over at least part of the length of an assigned screw shaft (14), wherein the clearing device (17) has a number of clearing tines (20) spaced apart from one another in the longitudinal direction of the screw shaft, wherein the clearing tines (20) are assigned with their free tine ends (26) to the assigned screw shaft (14), and wherein the raised screw helix (29) has in each region where a clearing tine (20) passes through the raised screw helix (29) a tine clearance (30), which is reached through by the respectively assigned clearing tine (20) during a rotation of the screw shaft.

2. Biogas plant according to Claim 1, **characterized in that** the tine clearance (30) on the screw helix is formed in such a way that it is reached through by the respectively assigned clearing tine (20) during a rotation of the screw shaft, in a shape- and/or contour-adapted manner with a defined gap distance.

3. Biogas plant according to Claim 1 or 2, **characterized in that** the at least one clearing tine (20) is assigned with its free tine end (26) to the assigned screw shaft (14) in such a way that the free tine end (26) lies at least partly in contact there or the free tine end (26) is at a defined gap distance (s) from the screw shaft (14) there.

4. Biogas plant according to Claim 3, **characterized in that**, preferably seen in the direction of rotation of the screw shaft (14), the gap distance (s) between the tine end (26) and the screw shaft (14) increases, in particular widens in sickle-shaped manner.

5. Biogas plant according to one of the preceding claims, **characterized in that** the at least one clearing tine (20) is formed adjustably in its distance from the assigned screw shaft (14) and/or in its length, in particular in such a way that the at least one clearing tine (20) is displaceable in a direction towards the assigned screw shaft (14) or away from it for setting a defined gap dimension (s) between the at least one clearing tine (20) and the assigned screw shaft (14).

6. Biogas plant according to one of the preceding claims, **characterized in that** at least one of the peripheral edges (31) of the screw helix (29) forming the tine clearance (30) and/or the tine end (26) of the clearing tine (20) assigned to the respective tine clearance (30) has and/or forms at least one cutting and/or striking edge (32, 33).

7. Biogas plant according to Claim 6, **characterized in that** the at least one cutting and/or striking edge (32, 33) is formed in a wedge-shaped and/or sharply tapering manner and/or is formed by a stepped and/or indented surface structure.

8. Biogas plant according to Claim 6 or 7, **characterized in that** the at least one cutting and/or striking edge (32, 33) on the at least one peripheral edge (31) of a tine clearance (30) and/or at a tine end (26) of the respectively assigned clearing tine (20) is or are aligned substantially transversely in relation to the axis of rotation (24) of the at least one screw shaft (14).

9. Biogas plant according to one of the preceding claims, **characterized in that** the feeding device has at least one charging opening (34), which is formed on the extruder barrel (16) and via which the biomass to be extruded can be fed to the cell breakdown extruder (6), wherein it is preferably provided that above the charging opening (34) there is arranged a charging shaft (37), which preferably has an upper charging funnel (38) and a shaft tube (39) adjoining the charging funnel (38) downwards towards the charging opening (34) and conically widening downwards, preferably with an inspection opening (40).

10. Biogas plant according to Claim 9, **characterized in that** at least one clearing device (17), and consequently the at least one clearing tine (20), is arranged in a region of the screw shaft that is facing the charging opening (18).

11. Biogas plant according to Claim 9 or 10, **characterized in that**, in the region of the charging opening (34), in particular on a peripheral edge of the charging opening (34) and/or on a region of the peripheral edge of the charging shaft (37) that is facing the charging opening (34) and/or at least on a front part of the charging opening (34) that is downstream as seen in the conveying direction, a directing element (41) is arranged, preferably in the form of a prism, which extends substantially in the transverse direction and/or over a defined part in the region of the charging opening (34).

12. Biogas plant according to one of Claims 9 to 11, **characterized in that** the at least one clearing tine (20) is offset downwards away from the charging opening (34), as seen in the direction of the vertical axis, in such a way that it is at a defined minimum distance from the charging opening (34), as seen in the direction of the vertical axis.

13. Biogas plant according to one of the preceding claims, **characterized in that** the at least one clearing tine (20) extends, at least with a part having the free tine end (26), away from the screw shaft (14) approximately as a straight extension along the screw shaft radius (r) taken from the shaft axis of rotation (24).

14. Biogas plant according to one of the preceding claims, **characterized in that** the cell breakdown extruder (6) is a twin-screw extruder with two screw shafts (14), wherein it is preferably provided that, with screw shafts (14) lying next to one another in the transverse direction, at least one clearing device (17) respectively extends with its respectively at least one clearing tine (20) inwards in the direction of one of the two screw shafts (14) from barrel walls (21, 22), in particular barrel side walls, lying opposite one another with respect to the screw shafts (14) lying next to one another.

15. Biogas plant according to Claim 14, **characterized in that**, in the intermeshing region (27) of the two screw shafts (14) rotating towards one another, arranged on at least one side of this intermeshing region (27) are directing elements (28) of the barrel, with which the biomass to be extruded can be directed in the direction of the intermeshing region (27) of the two screw shafts (14) or away from it, depending on the position and direction of rotation.

16. Biogas plant according to one of the preceding claims, **characterized in that** the at least one clearing device (17) is integrally or detachably connected to the extruder barrel (16), in particular has a carrier plate (23), which can be detachably connected to the inner wall of the barrel and from which the at least one clearing tine (20) protrudes.

## Revendications

1. Installation de biogaz comprenant un dispositif de chargement de fermenteur (1a) destiné à amener de la biomasse jusque dans un récipient de fermenteur (1b) de l'installation de biogaz (1), le dispositif de chargement de fermenteur (1a) comportant une extrudeuse de désagrégation de cellules (6) qui comporte au moins un arbre de vis sans fin (14) logé dans un boîtier d'extrudeuse (16) et pourvu d'une hélice de vis sans fin surélevée (29), et l'extrudeuse de désagrégation de cellules (6) comportant au moins un dispositif d'amenée de la biomasse à extruder et au moins une ouverture de sortie (18) destinée à la biomasse extrudée,
**caractérisée en ce que**,
au moins un dispositif de dégagement (17), qui s'étend sur au moins une partie de la longueur d'un arbre de vis sans fin (14) associé, est disposé dans l'espace intérieur du boîtier d'extrudeuse (16), le dispositif de dégagement (17) comportant une pluralité de dents de dégagement (20) espacées les unes des autres dans la direction longitudinale de l'arbre de vis sans fin, les dents de dégagement (20) étant associées, avec leurs extrémités de dents libres (26), à l'arbre de vis sans fin (14) associé, et l'hélice de vis sans fin surélevée (29) comportant, dans chaque zone de passage d'une dent de dégagement (20) ménagée à travers l'hélice de vis sans fin surélevée (29), un évidement de dent (30) à travers lequel la dent de dégagement (20) respective associée s'engage lorsque l'arbre de vis sans fin tourne.

2. Installation de biogaz selon la revendication 1, **caractérisée en ce que** l'évidement de dent (30) côté hélice de vis sans fin est conçu de manière que les dents de dégagement (20) respectives associées s'engagent à travers celui-ci de manière adaptée en forme et/ou contour avec un espacement défini lorsque l'arbre de vis tourne.

3. Installation de biogaz selon la revendication 1 ou 2, **caractérisée en ce que** l'au moins une dent de dégagement (20) est associée, avec son extrémité de dent libre (26), à l'arbre de vis sans fin (14) associé de sorte que l'extrémité de dent libre (26) vienne en appui au moins partiellement sur celui-ci ou l'extrémité de dent libre (26) présente à cet endroit un espacement défini (s) par rapport à l'arbre de vis sans fin (14).

4. Installation de biogaz selon la revendication 3, **caractérisée en ce que** l'espacement (s) ménagé entre l'extrémité de dent (26) et l'arbre de vis sans fin (14), de préférence vu dans le sens de rotation de l'arbre de vis sans fin (14), augmente, en particulier s'élargit en forme de faucille.

5. Installation de biogaz selon l'une des revendications précédentes, **caractérisée en ce que** l'au moins une dent de dégagement (20) est conçue pour être réglable en termes de distance de celle-ci à l'arbre de vis sans fin (14) associé et/ou en termes de longueur de celle-ci, notamment pour pouvoir approcher ou éloigner l'au moins une dent de dégagement (20) de celui-ci dans la direction de l'arbre de vis sans fin (14) associé afin de régler un espacement défini (s) entre l'au moins une dent de dégagement (20) et l'arbre de vis sans fin (14) associé.

6. Installation de biogaz selon l'une des revendications précédentes, **caractérisée en ce qu'**au moins un des bords (31), formant l'évidement de dent (30), de l'hélice de vis sans fin (29) et/ou l'extrémité de dent (26) de la dent de dégagement (20) associée à l'évidement de dent (30) respectif comportent et/ou forment au moins un bord de coupe et/ou de frappe (32, 33).

7. Installation de biogaz selon la revendication 6, **caractérisée en ce que** l'au moins un bord de coupe et/ou de frappe (32, 33) est en forme de coin et/ou est effilé en pointe et/ou est formé par une structure de surface étagée et/ou fracturée.

8. Installation de biogaz selon la revendication 6 ou 7, **caractérisée en ce que** l'au moins un bord de coupe et/ou de frappe (32, 33) sur l'au moins un bord (31) d'un évidement de dent (30) et/ou sur une extrémité de dent (26) de chaque dent de dégagement (20) associée et ou sont orienté(s) sensiblement transversalement à l'axe de rotation (24) de l'au moins un arbre de vis sans fin (14).

9. Installation de biogaz selon l'une des revendications précédentes, **caractérisée en ce que** le dispositif d'amenée comporte au moins une ouverture d'introduction (34) qui est formée au niveau du boîtier d'extrudeuse (16) et par laquelle la biomasse à extruder peut être amenée à l'extrudeuse de désagrégation de cellule (6), un conduit d'introduction (37) étant de préférence prévu qui est disposé au-dessus de l'ouverture d'introduction (34), qui comporte de préférence un entonnoir d'introduction supérieur (38) et un tube de conduit (39), pourvu de préférence d'une ouverture d'inspection (40), s'élargissant en cône vers le bas et se raccordant vers le bas à l'entonnoir d'introduction (38) en direction de l'ouverture d'introduction (34).

10. Installation de biogaz selon la revendication 9, **caractérisée en ce que** l'au moins un dispositif de dégagement (17), et donc l'au moins une dent de dégagement (20), est disposé dans une zone d'arbre de vis sans fin dirigée vers l'ouverture d'introduction (18).

11. Installation de biogaz selon la revendication 9 ou 10, **caractérisée en ce qu'**un élément de guidage (41), se présentant de préférence sous la forme d'un prisme, est disposé dans la zone de l'ouverture d'introduction (34), en particulier au niveau d'un bord de l'ouverture d'introduction (34) et/ou d'une zone de bord, dirigée vers l'ouverture d'introduction (34), du conduit d'introduction (37) et/ou d'une partie avant de l'ouverture d'introduction (34) par référence au sens de transport, lequel élément de guidage s'étend sensiblement dans la direction transversale et/ou sur une partie définie de la zone de l'ouverture d'introduction (34).

12. Installation de biogaz selon l'une des revendications 9 à 11, **caractérisée en ce que** l'au moins une dent de dégagement (20) est décalée vers le bas de l'ouverture d'introduction (34) par référence à la direction de l'axe vertical, et **en ce qu'**elle est située à une distance minimale définie de l'ouverture d'introduction par référence à l'axe vertical (34).

13. Installation de biogaz selon l'une des revendications précédentes, **caractérisée en ce que** l'au moins une dent de dégagement (20), au moins avec une partie de zone comportant l'extrémité de dent libre (26), s'écarte de l'arbre à vis sans fin (14) approximativement suivant une extension droite le long du rayon d'arbre de vis sans fin (r), partant de l'axe de rotation d'arbre (24).

14. Installation de biogaz selon l'une des revendications précédentes, **caractérisée en ce que** l'extrudeuse de désagrégation de cellule (6) est une extrudeuse à double vis sans fin comprenant deux arbres à vis sans fin (14), des parois de boîtier (21, 22) opposées par rapport aux arbres de vis sans fin (14) adjacents, en particulier des parois de boîtier latérales dans le cas d'arbres de vis sans fin (14) adjacents dans la direction transversale, étant de préférence prévues depuis lesquelles au moins un dispositif de dégagement (17), pourvu de sa au moins une dent de dégagement respective (20), s'étend vers l'intérieur en direction de l'un des deux arbres de vis sans fin (14).

15. Installation de biogaz selon la revendication 14, **caractérisée en ce que** des éléments de guidage côté boîtier (28) sont disposés dans la zone d'engagement (27) des deux arbres à vis sans fin (14) en rotation l'un par rapport à l'autre sur au moins un côté de cette zone d'engagement (27) et permettent, en fonction de la position et du sens de rotation, le guidage de la biomasse à extruder en direction de la zone d'engagement (27) des deux arbres de vis sans fin (14) ou dans la direction opposée.

16. Installation de biogaz selon l'une des revendications précédentes, **caractérisée en ce que** l'au moins un dispositif de dégagement (17) est relié d'une seule pièce ou de manière amovible au boîtier d'extrudeuse (16) et comporte notamment une plaque de support (23) qui peut être reliée de manière amovible à la paroi de boîtier intérieure et de laquelle l'au moins une dent de dégagement (20) fait saillie.
